# EUROPEAN PATENT SPECIFICATION

(11) **EP 1 827 335 B1**
(45) Date of publication and mention of the grant of the patent: **27.06.2012**
(21) Application number: 04809155.7
(22) Date of filing: 23.12.2004
(51) Int. Cl.: A61F 13/15

(54) **ABSORBENT ARTICLE**
SAUGFÄHIGER ARTIKEL
ARTICLE ABSORBANT

(43) Date of publication of application: 05.09.2007
(73) Proprietor: SCA Hygiene Products AB, 405 03 Göteborg (SE)
(72) Inventor: HANSSON, Morgan, SE-41451 Göteborg (SE); CORNELIUSSON, Helena, S-445 34 Bohus (SE)
(74) Representative: VALEA AB
(86) International application number: PCT/SE2004/001982
(87) International publication number: WO 2006/068549

(56) References cited:
- WO-A1-2004/062542
- US-A- 5 792 130
- US-A1- 2004 243 087
- US-B1- 6 328 724

## Description

### Technical field

The present invention refers to an absorbent article, such as a diaper, pant diaper, incontinent brief, sanitary napkin and the like intended to absorb and retain body fluid of the wearer.

### Background of the invention

Absorbent articles of the above mentioned kind are known, which have an absorbent core provided with folding guides in the crotch region, said folding guides dividing the crotch region of the absorbent core in a central portion and two lateral portions as seen in a transverse direction. These lateral portions can be folded with respect to the central portions so as to improve the fit and sealing effect against leakage.

WO 05/16418 discloses an absorbent core in an absorbent article comprising a central portion having a leg recess at each side thereof and two lateral portions placed laterally outside the central portion on respective sides thereof and in the respective leg recess. There is a gap between the central portion and the lateral portions and the outer and inner cover sheets are joined to each other in the gaps between the central and lateral portions. Contraction of the leg elastics of the diaper will cause the lateral portions to swing upwards and thus create a basin shape formed by the central portion and the upraised lateral portions.

US 5,382,246 discloses an absorbent article in the form of a diaper having an absorbent core comprising a central absorber and two peripheral absorbers, wherein the central absorber is separated from the peripheral absorbers by longitudinal slots. Expandable elastic strips fastened to the backsheet extend in these slots. The peripheral absorbers are located outside the expandable elastic strips. The elastic strips fit the bases of the thighs to bring the central absorber into close contact with the pubic region, while the peripheral absorbers are brought into close contact with the inside of the thighs.

EP-A-1 346 713 discloses a diaper having an absorbent core with lateral wings at the front and rear portion of the core, said wings being separated from the rest of the core by folding guides. Stretchable elastic members extend in the longitudinal direction outwardly of the folding guides and are at their ends attached to the direction outwardly of the folding guides and are at their ends attached to the wings and will fold the wings inwardly along the folding lines and bring them in close contact with the wearer's body.

US 2004/0123732 discloses a disposable diaper having an absorbent core comprising a pair of folding guides dividing the absorbent core in a central portion and a pair of lateral portions. Crotch elastics extend entirely outside the folding guides and cause the lateral portions to fold downwards to seal against the wearer's thighs.

Absorbent articles having an improved comfort by narrowing the width of the area where the article is applied in the crotch of the wearer (crotch area) during use are known through a number of prior art documents, for example EP-A-1 384 459. However if the width of the crotch portion is too narrow, the absorption capacity in this area, where the body fluid is discharged, will be insufficient and leakage is likely to occur.

### Object and most Important features of the invention

One object of the present invention is to provide an absorbent article which has an improved comfort and fit in the crotch region and which in use position has a narrow width in the crotch region, while at the same time maintaining a sufficient absorption capacity and sealing effect against leakage in this region.

Another object is to provide an absorbent article having an absorbent core which is formed into a basin-shape for improved liquid collecting capacity and reduced leakage risk.

These and further objects have according to the invention been achieved by an absorbent article comprising an absorbent core being provided with at least two folding guides extending in a substantial longitudinal direction in the crotch region and dividing at least a part of the crotch region of the absorbent core in a central portion and two lateral portions as seen in a transverse direction, wherein at least two stretchable crotch elastic members are arranged in the crotch portion of the article and are attached to the absorbent core and/or to one of the inner and outer covers, wherein at least a substantial portion of said crotch elastic members are positioned laterally outside the respective folding guide and another portion is located laterally inside the respective folding guide, so that the crotch elastic members cross the respective folding guide so as to cause said two lateral portions of the absorbent core to be raised along said folding guides with respect to the central portion.

According to one embodiment said folding guides have a curved shape.

In one aspect of the invention both ends of said folding guides extend to the adjacent longitudinal edge of the absorbent core, so that the at least two lateral portions are separated from the central portion of the absorbent core by the folding guides.

In a further embodiment the folding guides are substantially straight.

In one aspect of the invention one or both ends of the folding guides terminate inside the adjacent longitudinal edge of the absorbent core.

According to one embodiment the folding guides are grooves having a lower basis weight than the surrounding areas of the absorbent core. Alternatively the folding guides are formed by slits in the absorbent core. According to one embodiment said folding guides are grooves or slits extending through at least half the thickness of the absorbent core, preferably through the entire thickness of the absorbent core.

The folding guides are either continuous or intermittent slits or grooves.

The folding guides have length of at least 40 mm, preferably at least 50 mm

In one aspect of the invention the said crotch elastic members have an active region in longitudinal direction which is at least 40%, preferably at least 70%, of the extension in longitudinal direction of the respective folding guide. According to one embodiment the crotch elastic members have an active region in longitudinal direction which is at least equal to the entire extension in longitudinal direction of the folding guides.

The term "active region" is this respect refers to the distance in longitudinal direction (y) between the outer ends of the crotch elastic members, wherein an intermediate part of the respective elastic member may or may not be inactivated or simply cut away. Such an inactivated or absent intermediate portion of an elastic member is included in the active region, which is measured between the outer ends of the active parts of the respective elastic member.

In one aspect of the invention the crotch elastic members are positioned along or laterally outside of the lateral portions of the absorbent core over at least 75% of the length of the respective lateral portion in longitudinal direction.

According to one embodiment the crotch elastic members are positioned along the lateral portions of the absorbent core over at least 40%, preferably at least 50% of the length of the respective lateral portion in longitudinal direction.

The crotch elastic members extend in a straight or curved path.

In one embodiment the crotch elastic members are attached to the outer cover and/or the garment facing side of the absorbent core.

In a further aspect of the invention the invention is applied to an absorbent article in the form of a diaper, a pant diaper or an incontinence garment.

### Description of drawings

The invention will in the following be closer described with reference to some embodiments shown in the accompanying drawings.
Fig. 1 shows a simplified plan view of an absorbent article in the form of a diaper in its flat, uncontracted state.
Fig. 2 is a section according to the line II-II in Fig. 1.
Fig. 3 shows a perspective view of a diaper In a contracted state.
Fig. 4 is a section according to the line IV-IV in Fig. 3.
Fig. 5 shows a perspective view of a pant diaper from the front side.
Fig. 6 shows the pant diaper of Fig. 5 from the back side.
Fig. 7 is a section according to the line VII-VII in Fig. 5.
Fig. 8 is plan view of another embodiment In the form of an incontinence guard or sanitary napkin.
Fig. 9 is a section according to the line IX-IX in Fig. 8.
Fig. 10 illustrates how the crotch point of a wearer, an absorbent article and the corresponding absorbent core is determined.

### Definitions

### Absorbent article

The term "absorbent article" refers to products that are placed against the skin of the wearer to absorb and contain body exudates, like urine, faeces and menstrual fluid. The invention mainly refers to disposable absorbent articles, which are articles that are not intended to be laundered or otherwise restored or reused as an absorbent article after use.

### Inner liquid permeable cover

The inner liquid permeable cover forms the inner cover of the absorbent article and in use is placed in direct contact with the skin of the wearer. The inner liquid permeable cover can comprise a nonwoven material, e.g. spunbond, meltblown, carded, hydroentangled, wetlaid etc. Suitable nonwoven materials can be composed of natural fibers, such as woodpulp or cotton fibres, manmade fibres, such as polyester, polyethylene, polypropylene, viscose etc. or from a mixture of natural and manmade fibres. The inner liquid permeable cover material may further be composed of tow fibres, which may be bonded to each other in a bonding pattern, as e.g. disclosed in EP-A-1 035 818. Further examples of inner liquid permeable cover materials are porous foams, apertured plastic films etc. The materials suited as inner liquid permeable cover materials should be soft and non-irritating to the skin and be readily penetrated by body fluid, e.g. urine or menstrual fluid. The inner liquid permeable cover may further be different in different parts of the absorbent article.

### Outer liquid impermeable cover

The outer liquid impermeable cover forms the outer cover of the absorbent article at least on the core area thereof. The outer liquid impermeable cover can comprise a thin plastic film, e.g. a polyethylene or polypropylene film, a nonwoven material coated with a liquid impervious material, a hydrophobic nonwoven material, which resists liquid penetration, or a laminate, e.g. of a plastic film and a nonwoven material. The outer liquid impermeable cover material may be breathable so as to allow vapour to escape from the absorbent core, while still preventing liquids from passing through. Examples of breathable outer liquid impermeable cover materials are porous polymeric films, nonwoven laminates from spunbond and meltblown layers, laminates from porous polymeric films and nonwoven materials.

### Absorbent core

The "absorbent core" is the absorbent structure disposed between the two covers of the absorbent article. The absorbent core 5 can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbent polymers in an absorbent core. Superabsorbent polymers are water-swellable, water-insoluble organic or Inorganic materials capable of absorbing at least about 20 times its weight and in an aqueous solution containing 0.9 weight percent of sodium chloride. Organic materials suitable for use as a superabsorbent material can include natural materials such as polysaccharides, polypeptides and the like, as well as synthetic materials such as synthetic hydrogel polymers. Such hydrogel polymers include, for example, alkali metal salts of polyacrylic acids, polyacrylamides, polyvinyl alcohol, polyacrylates, polyacrylamides, polyvinyl pyridines, and the like. Other suitable polymers include hydrolyzed acrylonitrile grafted starch, acrylic acid grafted starch, and isobutylene maleic anhydride copolymers and mixtures thereof. The hydrogel polymers are preferably lightly crosslinked to render the material substantially water Insoluble. Preferred superabsorbent materials are further surface crosslinked so that the outer surface or shell of the superabsorbent particle, fiber, flake, sphere, etc. possesses a higher crosslink density than the inner portion of the superabsorbent. The superabsorbent materials may be in any form suitable for use in absorbent composites including particles, fibers, flakes, spheres, and the like.

A high absorption capacity is provided by the use of high amounts of superabsorbent material. For an absorbent core comprising a matrix of hydrophilic fibers, such as cellulosic fibers, and superabsorbent material, the proportion of superabsorbent material is preferably between 10 and 90% by weight, more preferably between 30 and 70% by weight.

It is conventional In absorbent articles to have absorbent cores comprising layers of different properties with respect to liquid receiving capacity, liquid distribution capacity and storage capacity. The thin absorbent bodies, which are common in for example baby diapers and incontinence guards, often comprise a compressed mixed or layered structure of cellulosic fluff pulp and superabsorbent polymers. The size and absorbent capacity of the absorbent core may be varied to be suited for different uses such as for infants or for adult incontinent persons.

### Crotch point and crotch line

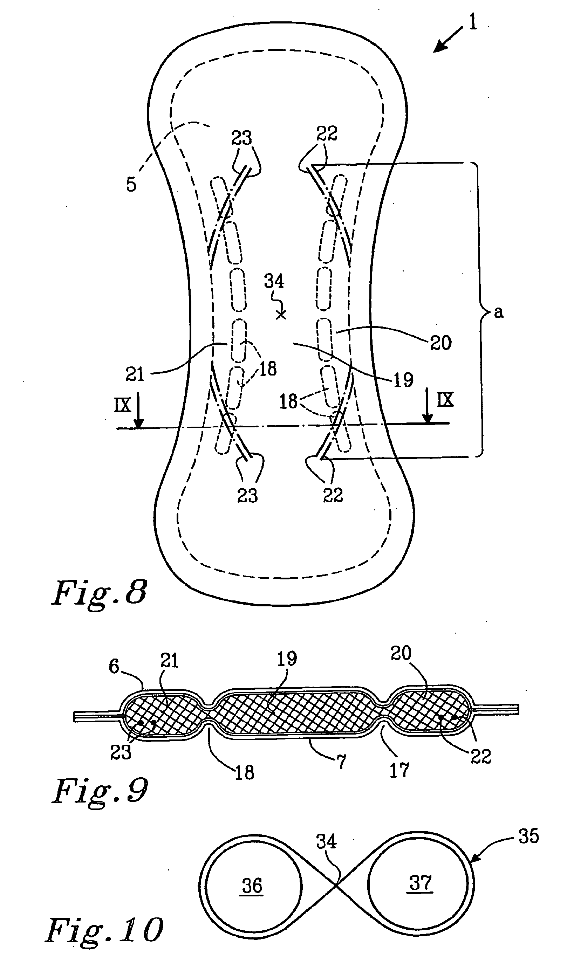

The crotch point of an absorbent article and of the absorbent core of an absorbent article as defined In for example EP-B1-0 969 784, i.e. it is determined by placing the article on a wearer in a standing position and then placing an extensible filament 31 around the legs 32, 33 in a figure eight configuration (Fig. 10). The point in the article and the absorbent core corresponding to the point of intersection of the filament is deemed to be the crotch point 34. It is understood that the crotch point is determined by placing the absorbent article on the wearer in the intended manner and determining where the crossed filament would contact the article/core. The crotch line is a line in the transverse direction, x, of the article/core extending through the crotch point 34.

### Crotch region

In a normal case the "crotch region" 12 is determined by first locating the crotch point 34 of the absorbent core, and then measuring forward and backward a distance of 25% of the total length of the core. In a case, in which the absorbent core is located asymmetrically in the article, for example the main part of the core is located towards the front of the article and the rear edge of the core is located a distance from the crotch point which is equal to or less than 25% of the total length of the core, the crotch region extends from the rear end of the core, to the crotch point 34 and a distance forwards which is 25% of the total length of the core, or vice versa in case the main part of the core is located towards the back of the article. Thus in the normal case the "crotch region" of the absorbent core corresponds to 50% of the total length of the absorbent core (i.e. in the longitudinal direction, y direction), where the crotch point 34 is located in the longitudinal center of the crotch region. For an asymmetrically placed core, as described above, the crotch region 12 may be shorter than 50% of the total length of the core and the crotch point 34 may be located outside the longitudinal center of the crotch region 12.

### Description of preferred embodiments

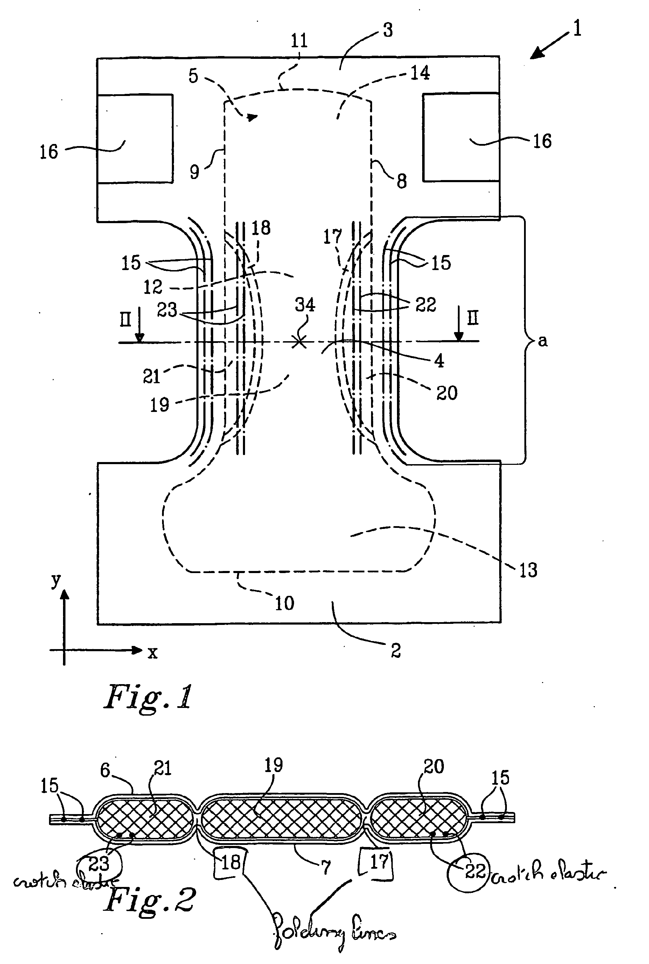

Figure 1 shows a simplified plan view of an absorbent article in a flat, uncontracted state. The absorbent article shown in Figure 1 is in the form of a diaper 1 having a longitudinal, y, and a transverse direction, x, and comprises, as seen in its longitudinal direction, a front portion 2, a back portion 3 and a crotch portion 4 there between. In Its most common form the diaper comprises an absorbent core 5 and a cover enclosing the absorbent core. Said cover comprises an inner liquid pervious cover 6 on the wearer facing side of the absorbent core 5 and an outer liquid impervious cover 7 on the garment facing side of the absorbent core. The inner liquid pervious cover 6 is often referred to as topsheet, while the outer liquid impervious cover 7 is often referred to as backsheet.

The absorbent core 5 has an elongated shape and comprises a pair of longitudinal edges 8, 9 and a pair of transverse edges 10, 11. The absorbent core 5 has a crotch region 12 and two outer regions 13, 14 as seen in the longitudinal direction, y, of the article. The outer regions 13, 14 are located adjacent the respective transverse edges 10, 11 of the core. The crotch region 12 is the region of the core as defined above. For an article having a core 5 which is placed asymmetrically in the article, the core may have only one outer region 13 or 14 and a crotch region 12, wherein one of the transverse edges 10 or 11 may form the termination of the crotch region 12 in one longitudinal direction.

The inner cover 6 and the outer cover 7 extend outward beyond the peripheral edges of the absorbent core 5 and have their inner surfaces bonded to each other, e g by gluing or welding by heat or ultrasonic. The inner and outer cover materials may further be bonded, e.g. by adhesive, to the absorbent core.

The areas of the article adjacent the leg openings are along the longitudinal side edges provided with elastic members 15 which are bonded between the inner cover 6 and the outer cover 7 material layers in a stretched condition so as to provide elasticized leg openings of the diaper. Corresponding elastic members (not shown) may be arranged to extend in the transverse, x, direction in the front 2 and back region 3 adjacent the transverse side edges forming the waist opening of the diaper.

The elastic members may alternatively be of a material that is activatable by some means, for example by heat, to an elastified state, wherein they may be attached to the article In an unstretched inactivated state and are subsequently activated to a contracted elastic state.

The back region 3 is provided with fasteners 16 attached thereto. The fasteners are intended to be fastened to the front region of the particle to form a pant-like shape. The fasteners 16 may be in the form of adhesive tapes or hook elements adapted to attach to a loop material, for example In the form of a nonwoven material forming the outer coversheet of the diaper.

The absorbent core 5 in Figures 1 and 2 is provided with two folding guides 17, 18 extending in a substantially longitudinal direction y in the crotch region 12 and dividing at least a part of the crotch region of the absorbent core 5 in a central portion 19 and two lateral portions 20, 21 as seen in a transverse direction x. The term "substantially longitudinal direction" in this context means that they have their longest extension in the longitudinal direction, y, as compared to the transverse direction, x. The folding guides 17, 18 may have a straight or curved shape.

The folding guides 17, 18 may be slits or grooves extending through the entire or part of the thickness of the absorbent core. The grooves may have a lower basis weight than the surrounding areas of the absorbent core, said grooves having a certain width of for example a few millimeters, or they may be cuts or slits made in the absorbent core. Preferably they extend through at least half the thickness of the absorbent core. In a case where the absorbent core comprises two or more layers, the slits or grooves may extend through one, more or all layers. In a case where they do not extend through the entire thickness of the absorbent core they may extend from the side of the absorbent core 5 facing the outer cover 7 or from the side facing the inner cover 6.

The inner 6 and outer cover 7 materials may be joined to each other through the folding guides 17, 18 in case these extend through the entire thickness of the absorbent core 5, but the inner and outer covers may alternatively be free from each other in the area of the folding guides.

In a preferred embodiment the folding guides 17, 18 have an extension in the longitudinal direction, y, of the article that does not go beyond the crotch region 12 of the absorbent core 5.

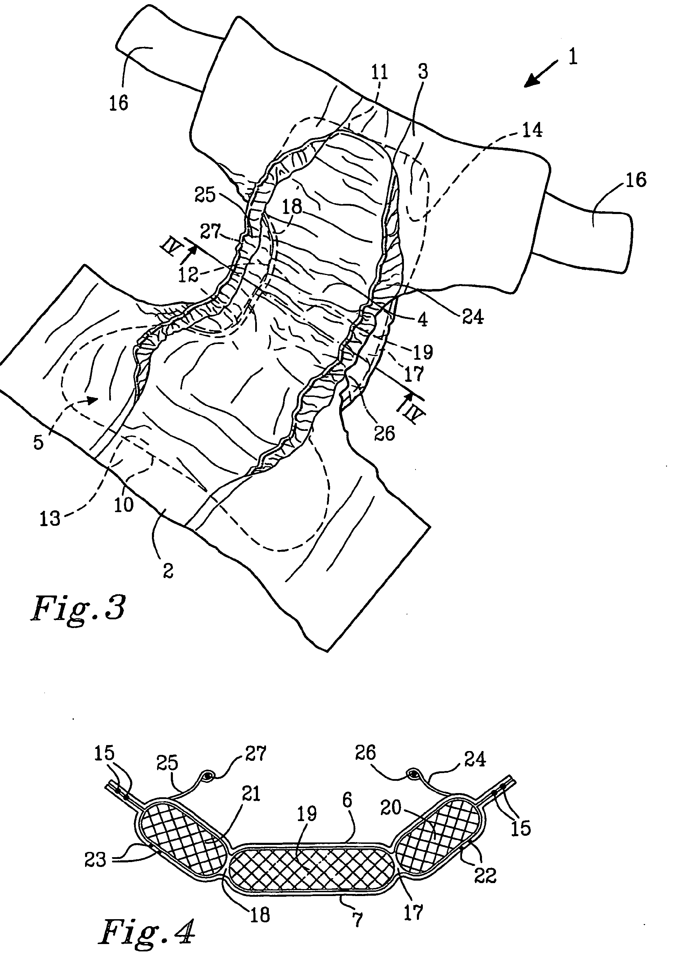

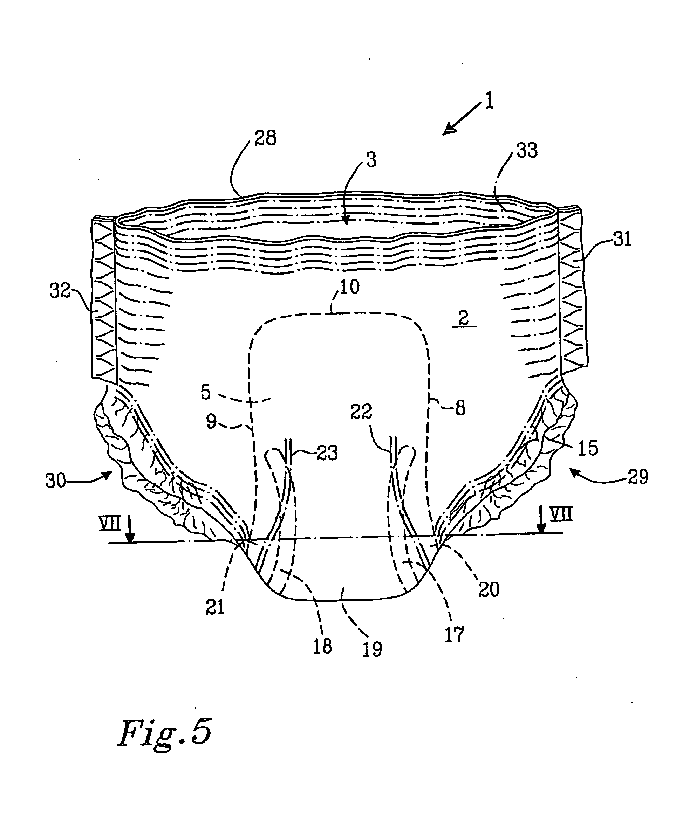

In Figures 1 it is shown that both ends of the folding guides 17, 18 extend all the way to the adjacent longitudinal edge 8, 9 of the absorbent core 5, so that the two lateral portions 20, 21 are separated from the central portion 19 of the absorbent core by the folding guides. This applies also to the embodiment in Fig. 3, while in the embodiment of Figures 5, 6 and 8 both ends of the folding guides 17, 18 terminate inside the adjacent longitudinal edge 8, 9 of the absorbent core 5. The folding guides 17, 18 may of course alternatively have one end extending all the way to the adjacent longitudinal edge of the absorbent core and one end terminating inside thereof.

The length of the folding guides 17, 18 may vary depending on the type and size of the absorbent article, for example whether the article is intended for babies or for incontinent adults. The length of the slits or grooves is however at least 40 mm, preferably at least 50 mm. In a case where the slits or grooves are intermittent or "broken", i.e. consist of a row of two or more short slits or grooves, as is shown in Figure 8, the length is defined as the total length of the row of short slits or grooves.

A pair of stretchable crotch elastic members 22, 23 is arranged in the crotch portion 4 of the article and are contractably attached to the absorbent core 5 and/or to one of the Inner or outer covers 6 or 7. Alternatively they are of a material that is activatable by some means, for example by heat, to an elastifled state, wherein they may be attached to the article in an unstretched inactivated state and are subsequently activated to a contracted elastic state.

The term "attached to the absorbent core" means that the crotch elastic members 22, 23 are either attached to the garment or wearer facing side of the absorbent core 5 or within the absorbent core 5, between layers thereof. Thus the crotch elastic members 22, 23 can either be attached to the inner or outer covers 6, 7 only, to one of the inner or outer covers and to the absorbent core 5 or within the absorbent core 5, between layers thereof. Preferably the crotch elastic member 22, 23 are attached to the outer cover 7 and/or to the garment facing side of the absorbent core. These crotch elastic members 22, 23 may be straight or curved and have an extension in longitudinal direction, y, as well as in a transverse direction, x. At least a substantial portion of said crotch elastic members 22, 23, which means at least 50%, preferably at least 75% of their length, is positioned laterally outside the respective folding guide 17, 18. Contraction of the crotch elastic member 22, 23 will cause the two lateral portions 20, 21 of the absorbent core 5 to be raised along the folding guides 17, 18 with respect to the central portion 19.

In the embodiment shown in Figure 1 the crotch elastic member 22, 23 extend along a substantially straight path substantially in parallel to the longitudinal direction, y. They cross the respective folding guide 17, 18 close to the ends of the folding guides and terminate a short distance longitudinally outside the folding guides. It is preferred that the crotch elastic members 22, 23 have an active region in longitudinal direction which is at least 40%, preferably at least 70%, of the extension of the folding guides in longitudinal direction, preferably an active region which is at least 100% of the extension in longitudinal direction of the folding guides 17, 18, in order to effectively raise the lateral portions 20 and 21 of the absorbent core 5.

The term "active region" is herewith defined as above, i.e. the distance, a, in longitudinal direction, y, between the outer ends of the crotch elastic members 22, 23, wherein an intermediate part of the respective elastic member may or may not be inactivated or simply cut away. Such an inactivated or absent intermediate portion of an elastic member is included in the active region, which is measured between the outer ends of the active parts of the respective elastic member. This is shown in Figure 8, wherein an intermediate part of the crotch elastic members 22, 23, which otherwise would have been located outside the absorbent core 5, are cut away.

It is further preferred that the crotch elastic members 22, 23 are positioned on, in or laterally outside the lateral portions 20, 21 of the absorbent core 5 over at least 75% of the length of the respective lateral portion in longitudinal direction y. Preferably the crotch elastic members 22, 23 are positioned on or in the lateral portions 20, 21 of the absorbent core 5 over at least 40%, preferably at least 50% of the length of the respective lateral portion in longitudinal direction y.

The raising of the lateral portions 20, 21 is illustrated in Figures 3 and 4 showing a diaper in a contracted shape, when the lateral portions 20, 21 have been folded upwards by the crotch elastic members 22, 23. The diaper further is provided with elastic barrier flaps 24, 25 having a proximal edge and a distal edge, and an elastic member 26, 27 contractably attached at the respective distal edge, thus spacing the distal edge of the barrier flaps 24, 25 from the inner cover 6. These barrier flaps 24, 25 form leakage barriers and are at their proximal edge secured to the inner cover 6 outside or above the absorbent core 5.

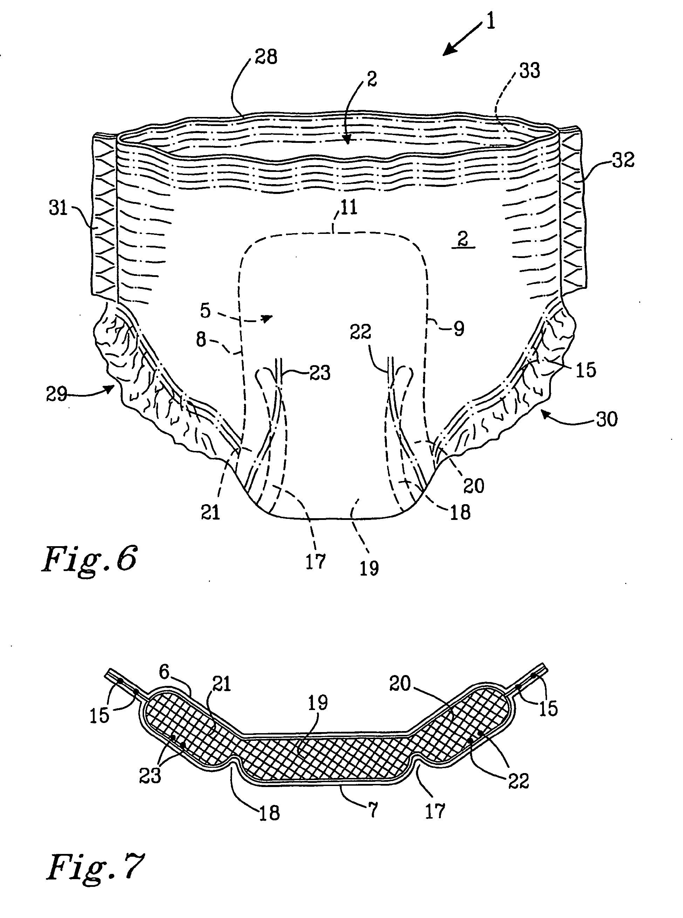

Figure 5 and 6 shows a so called pant diaper In which the front and back regions 2 and 3 are joined to each other along their longitudinal side edges thereof forming side seams 31, 32 to define a waist-opening 28 and a pair of leg-openings 29, 30. The front and back regions 2 and 3 are joined along said side seams 31 and 32, for example by adhesive, ultrasonic welding, heat sealing or the like. The front and back regions 2 and 3 can either be joined along said side seams with the inner cover 6 facing inwards, as is shown in the drawings. Alternatively they are joined in an overlapped manner with the inner cover 6 of either the front or back region facing the outer cover 7 of the opposite region.

In an alternative form the pant diaper comprises a core region comprising the absorbent core, a liquid pervious inner cover and a liquid impervious outer cover as disclosed above, and a chassis region outside the core region, wherein the chassis region comprises a coversheet of a soft and comfortable material, for example an elastic laminate. An example of such a pant diaper is disclosed in PCT/SE2004/001004.

The waist area, at least a part of the leg opening area and the side areas adjacent the side seams 31 and 32 are elasticized. The elastification is usually accomplished by a plurality of elastic members, such as elastic threads 33, contractably affixed In a stretched condition between the outer cover 7 and the inner cover 6. Alternatively the elastic members 33 are of a material that is activatable by some means, for example by heat, to an elastified state, wherein they may be attached to the article in an unstretched inactivated state and are subsequently activated to a contracted elastic state.

In a further alternative elastic materials, such as an elastic laminate, may be used to form the chassis of the article in those areas where elasticity is desired.

Figure 5 shows the pant diaper from the front side while Figure 6 shows the same pant diaper from the rear side. The folding guides 17, 18 are slits or grooves that do not extend through the entire thickness of the absorbent core. They do further not extend all the way out to the respective longitudinal edge 8 and 9 of the absorbent core 5, but terminate a short distance inside thereof. The crotch elastic members 22, 23 extend along a curved path crossing the folding guides 17, 18 close to their ends.

It is illustrated how the crotch elastic members 22, 23 will lift the lateral portions 20, 21 of the absorbent core along the folding guides 17, 18 to form a narrow crotch width of the absorbent core. At the same time the absorbent core 5 forms a "basin"-shape in the crotch region, where the central portion 19 of the absorbent core forms the bottom of the "basin" and the lateral portions 20, 21 form the upraised side portions of the "basin".

Figure 8 shows a further embodiment in the form of an incontinence guard or sanitary napkin intended to be worn in the panties of a wearer. The folding guides 17, 18 are in the form of intermittent or "broken" slits and the crotch elastic members 22, 23 extend along a curved path. The absorbent core 5 is in Figure 9 shown as a multi-layered core and the crotch elastic members 22, 23 are attached between two layers of the absorbent core. Figures 8 and 9 show the article In a flat uncontracted state, but it is understood that in its contracted state the crotch elastic members 22, 23 will lift the lateral portions 20, 21 upwards along the folding guides 17, 18.

As mentioned above an intermediate part of the crotch elastic members 22, 23, which otherwise would have been located outside the absorbent core 5, are cut away. These absent intermediate portions of the elastic member 22, 23 are included in the active region, a, which is measured between the outer ends of the active parts of the respective elastic member.

Figure 10 illustrates how the crotch point 34 of an absorbent article and of the absorbent core of an absorbent article is determined by placing the article on a wearer in a standing position and then placing an extensible filament 35 around the legs 36, 37 in a figure eight configuration.

It is understood that the configuration of the folding guides, 17, 18 and crotch elastic member 22, 23 may be varied and combined in many ways and that details from the different embodiments described above and shown in the drawing may be exchanged and combined in an optional way. It would also be feasible to have folding guides 17, 18 comprising two or more slits arranged substantially in parallel close to each other.

## Claims

1. An absorbent article comprising an absorbent core (5) and a cover enclosing the absorbent core, said cover comprising a liquid pervious inner cover (6) on the wearer facing side of the absorbent core and a liquid impervious outer cover (7) on the garment facing side of the absorbent core, said particle having a longitudinal (y) and a transverse (x) direction and comprises as seen in its longitudinal direction a front portion (2), a back portion (3) and a crotch portion (4) there between, said absorbent core (5) having an elongated shape and comprises a pair of longitudinal edges (8, 9) and a front (10) and a back transverse edge (11), said core having a crotch region (12) and at least one outer region (13; 14) as seen in the longitudinal direction (y) of the article, said outer region(-s) being located adjacent the respective transverse edge (10, 11) of the absorbent core (5), said absorbent core further being provided with at least two folding guides (17,18) extending in a substantially longitudinal direction (y) in said crotch region and dividing at least a part of the crotch region of the absorbent core in a central portion (19) and two lateral portions (20, 21) as seen in a transverse direction, said folding guides (17, 18) are grooves or slits extending through at least half the thickness of the absorbent core (5),
**characterized in**
**that** at least two stretchable crotch elastic members (22, 23) are arranged in the crotch portion (4) of the article and are attached to the outer cover (7) and/or to the garment facing side of the absorbent core (5), wherein at least a substantial portion of said crotch elastic members (22, 23) are positioned laterally outside the respective folding guide (17, 18) and another portion is located laterally inside the respective folding guide, so that the crotch elastic members cross the respective folding guide, so as to cause said two lateral portions (20, 21) of the absorbent core to be raised along said folding guides with respect to the central portion (19).

2. An absorbent article as claimed in claim 1,
**characterized in**
**that** said folding guides (17, 18) have a curved shape.

3. An absorbent article as claimed in claim 1 or 2,
**characterized in**
**that** both ends of said folding guides (17, 18) extend to the adjacent longitudinal edge (8, 9) of the absorbent core (5).

4. An absorbent article as claimed in claim 1,
**characterized in**
**that** said folding guides (17, 18) are substantially straight.

5. An absorbent article as claimed in claim 1, 2 or 4,
**characterized in**
**that** one or both ends of said folding guides (17, 18) terminate inside the adjacent longitudinal edge (8, 9) of the absorbent core (5).

6. An absorbent article as claimed in any of the preceding claims, **characterized in**
**that** said folding guides (17, 18) are grooves having a lower basis weight than the surrounding areas of the absorbent core.

7. An absorbent article as claimed in any of claims 1-5,
**characterized in**
**that** said folding guides (17, 18) are formed by slits in the absorbent core.

8. An absorbent article as claimed in claim 7,
**characterized in**
**that** said folding guides (17, 18) are grooves or slits extending through the entire thickness of the absorbent core (5).

9. An absorbent article as claimed in any of claims 6-8,
**characterized in**
**that** said folding guides (17, 18) are either continuous or intermittent slits or grooves.

10. An absorbent article as claimed in any of the preceding claims,
**characterized in**
**that** said folding guides (17, 18) have length of at least 40 mm, preferably at least 50 mm.

11. An absorbent article as claimed in any of the preceding claims,
**characterized in**
**that** said crotch elastic members (22, 23) have an active region in longitudinal direction which is at least 40%, preferably at least 70%, of the extension in longitudinal direction (y) of the respective folding guide (17, 18).

12. An absorbent article as claimed in claim 11,
**characterized in**
**that** said crotch elastic members (22, 23) have an active region in longitudinal direction which is at least equal to the entire extension in longitudinal direction (y) of the folding guides (17, 18).

13. An absorbent article as claimed in any of the preceding claims,
**characterized in**
**that** said crotch elastic members (22, 23) are positioned on, in or laterally outside the lateral portions (20, 21) of the absorbent core (5) over at least 75% of the length of the respective lateral portion in longitudinal direction (y).

14. An absorbent article as claimed in claim 13,
**characterized in**
**that** said crotch elastic members (22, 23) are positioned on or in the lateral portions (20, 21) of the absorbent core (5) over at least 40%, preferably at least 50% of the length of the respective lateral portion in longitudinal direction (y).

15. An absorbent article as claimed in any of claims 11-14,
**characterized in**
**that** said crotch elastic members (22, 23) extend in a straight or curved path.

16. An absorbent article as claimed in any of the preceding claims,
**characterized in**
**that** the article is a diaper, a pant diaper or an incontinence garment.

## Patentansprüche

1. Saugfähiger Artikel mit einem saugfähigen Kern (5) und einer den saugfähigen Kern umgebenden Abdeckung, die eine flüssigkeitsdurchlässige innere Abdeckung (6) an der dem Träger gegenüberstehenden Seite des saugfähigen Kerns und eine flüssigkeitsundurchlässige äußere Abdeckung (7) auf der der Bekleidung gegenüberstehenden Seite des saugfähigen Kerns aufweist, wobei der Artikel eine Längsrichtung (y) und eine Querrichtung (x) und in seiner Längsrichtung gesehen einen vorderen Abschnitt (2), einen hinteren Abschnitt (3) und dazwischen einen Schrittabschnitt (4) aufweist, der saugfähige Kern (5) eine längliche Form und ein Paar Längskanten (8, 9) und eine vordere (10) und eine hintere Querkante (11) aufweist, der Kern einen Schrittbereich (12) und mindestens einen in der Längsrichtung (y) des Artikels gesehenen äußeren Bereich (13; 14) aufweist, der äußere Bereich an die jeweilige Querkante (10, 11) des saugfähigen Kerns (5) angrenzend angeordnet ist, der saugfähige Kern des Weiteren mit mindestens zwei Faltführungen (17, 18) bereitgestellt ist, die sich in dem Schrittbereich im Wesentlichen in einer Längsrichtung (y) erstrecken und in Querrichtung gesehen zumindest einen Teil des Schrittbereichs des saugfähigen Kerns in einen mittleren Abschnitt (19) und zwei seitliche Abschnitte (20, 21) teilen und die Faltführungen (17, 18) Nuten oder Schlitze sind, die sich durch mindestens die Hälfte der Dicke des saugfähigen Kerns (5) erstrecken,
**dadurch gekennzeichnet, dass**
mindestens zwei dehnbare elastische Schrittelemente (22, 23) in dem Schrittabschnitt (4) des Artikels angeordnet sind und an der äußeren Abdeckung (7) und/oder an der der Bekleidung gegenüberliegenden Seite des saugfähigen Kerns (5) angebracht sind, wobei zumindest ein wesentlicher Abschnitt der elastischen Schrittelemente (22, 23) seitlich außerhalb der jeweiligen Faltführung (17, 18) angeordnet ist und ein anderer Abschnitt seitlich in der jeweiligen Faltführung angeordnet ist, sodass die elastischen Schrittelemente die jeweilige Faltführung schneiden, um zu verursachen, dass die zwei seitlichen Abschnitte (20, 21) des saugfähigen Kerns in Bezug zu dem mittleren Abschnitt (19) entlang der Faltführungen angehoben werden.

2. Saugfähiger Artikel nach Anspruch 1, **dadurch gekennzeichnet,**
**dass** die Faltführungen (17, 18) eine gekrümmte Form aufweisen.

3. Saugfähiger Artikel nach Anspruch 1 oder 2, **dadurch gekennzeichnet,**
**dass** sich beide Enden der Faltführungen (17, 18) zu der angrenzenden Längskante (8, 9) des saugfähige Kerns (5) erstrecken.

4. Saugfähiger Artikel nach Anspruch 1, **dadurch gekennzeichnet,**
**dass** die Faltführungen (17, 18) im Wesentlichen gerade sind.

5. Saugfähiger Artikel nach Anspruch 1, 2 oder 4, **dadurch gekennzeichnet,**
**dass** ein Ende oder beide Enden der Faltführungen (17, 18) in der angrenzenden Längskante (8, 9) des saugfähigen Kerns (5) enden.

6. Saugfähiger Artikel nach einem der vorstehenden Ansprüche, **dadurch gekennzeichnet,**
**dass** die Faltführungen (17, 18) Nuten sind, die ein geringeres Flächengewicht als die umgebenden Bereiche des saugfähigen Kerns aufweisen.

7. Saugfähiger Artikel nach einem der Ansprüche 1-5, **dadurch gekennzeichnet,**
**dass** die Faltführungen (17, 18) durch Schlitze in dem saugfähigen Kern ausgebildet sind.

8. Saugfähiger Artikel nach Anspruch 7, **dadurch gekennzeichnet,**
**dass** die Faltführungen (17, 18) Nuten oder Schlitze sind, die sich durch die gesamte Dicke des saugfähigen Kerns (5) erstrecken.

9. Saugfähiger Artikel nach einem der Ansprüche 6-8, **dadurch gekennzeichnet,**
**dass** die Faltführungen (17, 18) entweder durchgehende oder unterbrochene Schlitze oder Nuten sind.

10. Saugfähiger Artikel nach einem der vorstehenden Ansprüche, **dadurch gekennzeichnet,**
**dass** die Faltführungen (17, 18) eine Länge von mindestens 40 mm, bevorzugt mindestens 50 mm aufweisen.

11. Saugfähiger Artikel nach einem der vorstehenden Ansprüche, **dadurch gekennzeichnet,**
**dass** die elastischen Schrittelemente (22, 23) in Längsrichtung einen aktiven Bereich aufweisen, der mindestens 40%, bevorzugt mindestens 70% der Ausdehnung in Längsrichtung (y) der jeweiligen Faltführung (17, 18) ist.

12. Saugfähiger Artikel nach Anspruch 11, **dadurch gekennzeichnet,**
**dass** die elastischen Schrittelemente (22, 23) in Längsrichtung einen aktiven Bereich aufweisen, der zumindest gleich der gesamten Ausdehnung in der Längsrichtung (y) der Faltführungen (17, 18) ist.

13. Saugfähiger Artikel nach einem der vorstehenden Ansprüche, **dadurch gekennzeichnet,**
**dass** die elastischen Schrittelemente (22, 23) auf, in oder seitlich außerhalb der seitlichen Abschnitte (20, 21) des saugfähigen Kerns (5) über mindestens 75% der Länge des jeweiligen seitlichen Abschnitts in Längsrichtung (y) angeordnet sind.

14. Saugfähiger Artikel nach Anspruch 13, **dadurch gekennzeichnet,**
**dass** die elastischen Schrittelemente (22, 23) auf oder in den lateralen Abschnitten (20, 21) des saugfähigen Kerns (5) über mindestens 40%, bevorzugt mindestens 50% der Länge des jeweiligen seitlichen Abschnitts in Längsrichtung (y) angeordnet sind.

15. Saugfähiger Artikel nach einem der Ansprüche 11-14, **dadurch gekennzeichnet,**
**dass** sich die elastischen Schrittelemente (22, 23) in einem geraden oder gekrümmten Pfad erstrecken.

16. Saugfähiger Artikel nach einem der vorstehenden Ansprüche, **dadurch gekennzeichnet,**
**dass** der Artikel eine Windel, eine Hosenwindel oder eine Inkontinenzbekleidung ist.

## Revendications

1. Article absorbant, comprenant un corps absorbant (5) et un élément de recouvrement enfermant le corps absorbant, ledit élément de recouvrement étant constitué d'un élément de recouvrement intérieur (6) perméable aux liquides, situé sur la face côté utilisateur du corps absorbant, et d'un élément de recouvrement extérieur (7) imperméable aux liquides, situé sur la face côté vêtement du corps absorbant, ledit article ayant un sens longitudinal (y) et un sens transversal (x) et comportant, vu dans son sens longitudinal, une partie avant (2), une partie arrière (3) et une partie formant l'entrejambe (4) entre ces deux parties, ledit corps absorbant (5) ayant une forme allongée et comportant une paire de bords longitudinaux (8, 9) et un bord transversal avant (10) et un bord transversal arrière (11), ledit corps ayant une zone d'entrejambe (12) et au moins une zone extérieure (13; 14), vues dans le sens longitudinal (y) de l'article, ladite/lesdites zone(s) extérieure(s) étant située(s) à proximité du bord transversal (10, 11) respectif du corps absorbant (5), ledit corps absorbant étant en outre pourvu d'au moins deux guides de pliage (17, 18) s'étendant dans un sens essentiellement longitudinal (y) dans ladite zone d'entrejambe, et divisant au moins une partie de la zone d'entrejambe du corps absorbant en une partie centrale (19) et deux parties latérales (20, 21), vues dans une direction transversale, lesdits guides de pliage (17, 18) étant des rainures ou fentes s'étendant sur au moins la moitié de l'épaisseur du corps absorbant (5), **caractérisé en ce que**
au moins deux éléments élastiques d'entrejambe (22, 23) étirables sont disposés dans la partie d'entrejambe (4) de l'article et sont fixés à l'élément de recouvrement extérieur (7) et/ou à la face côté vêtement du corps absorbant (5), au moins une partie substantielle desdits éléments élastiques d'entrejambe (22, 23) étant placée latéralement à l'extérieur du guide de pliage (17, 18) respectif, et une autre partie étant placée latéralement à l'intérieur du guide de pliage respectif, de manière à ce que les éléments élastiques d'entrejambe croisent le guide de pliage respectif, de telle sorte que lesdites deux parties latérales(20, 21) du corps absorbant soient levées le long desdits guides, par rapport à la partie centrale (19).

2. Article absorbant selon la revendication 1, **caractérisé en ce que** lesdits guides de pliage (17, 18) ont une forme incurvée.

3. Article absorbant selon la revendication 1 ou 2, **caractérisé en ce que** les deux extrémités desdits guides de pliage (17, 18) s'étendent jusqu'au bord longitudinal (8, 9) adjacent du corps absorbant (5).

4. Article absorbant selon la revendication 1, **caractérisé en ce que** lesdits guides de pliage (17, 18) sont sensiblement rectilignes.

5. Article absorbant selon la revendication 1, 2 ou 4, **caractérisé en ce qu'**une extrémité ou les deux extrémités desdits guides de pliage (17, 18) se termine(nt) à l'intérieur du bord longitudinal (8, 9) adjacent du corps absorbant (5).

6. Article absorbant selon l'une quelconque des revendications précédentes, **caractérisé en ce que** lesdits guides de pliage (17, 18) sont des rainures ayant une masse surfacique de base plus faible que les zones environnantes du corps absorbant.

7. Article absorbant selon l'une quelconque des revendications 1 à 5, **caractérisé en ce que** lesdits guides de pliage (17, 18) sont formés par des fentes dans le corps absorbant.

8. Article absorbant selon la revendication 7, **caractérisé en ce que** lesdits guides de pliage (17, 18) sont des rainures ou fentes s'étendant sur toute l'épaisseur du corps absorbant (5).

9. Article absorbant selon l'une quelconque des revendications 6 à 8, **caractérisé en ce que** lesdits guides de pliage (17, 18) sont des fentes ou rainures continues ou intermittentes.

10. Article absorbant selon l'une quelconque des revendications précédentes, **caractérisé en ce que** lesdits guides de pliage (17, 18) ont une longueur d'au moins 40 mm, de préférence d'au moins 50 mm.

11. Article absorbant selon l'une quelconque des revendications précédentes, **caractérisé en ce que** lesdits éléments élastiques d'entrejambe (22, 23) comportent une zone active, dans le sens longitudinal, qui correspond à au moins 40 %, de préférence à au moins 70 %, de la dimension dans le sens longitudinal (y) du guide de pliage (17, 18) respectif.

12. Article absorbant selon la revendication 11, **caractérisé en ce que** lesdits éléments élastiques d'entrejambe (22, 23) comportent une zone active, dans le sens longitudinal, qui correspond au moins à la dimension totale dans le sens longitudinal (y) des guides de pliage (17, 18).

13. Article absorbant selon l'une quelconque des revendications précédentes, **caractérisé en ce que** lesdits éléments élastiques d'entrejambe (22, 23) sont disposés sur ou dans les parties latérales (20, 21) du corps absorbant (5) ou latéralement à l'extérieur de celles-ci, sur au moins 75 % de la longueur de la partie latérale respective, dans le sens longitudinal (y).

14. Article absorbant selon la revendication 13, **caractérisé en ce que** lesdits éléments élastiques d'entrejambe (22, 23) sont disposés sur ou dans les parties latérales (20, 21) du corps absorbant (5), sur au moins 40 %, de préférence au moins 50 % de la longueur de la partie latérale respective, dans le sens longitudinal (y).

15. Article absorbant selon l'une quelconque des revendications 11 à 14, **caractérisé en ce que** lesdits éléments élastiques d'entrejambe (22, 23) s'étendent dans un plan rectiligne ou incurvé.

16. Article absorbant selon l'une quelconque des revendications précédentes, **caractérisé en ce que** l'article est une couche, une couche-culotte ou un vêtement pour incontinence.
